# EUROPÄISCHE PATENTANMELDUNG

(11) **EP 4 549 427 A1**
(43) Veröffentlichungstag der Anmeldung: **07.05.2025**
(21) Anmeldenummer: 23207657.0
(22) Anmeldetag: 03.11.2023
(51) Int. Cl.: C07C 67/38, C07C 69/753

(54) **ALKOXYCARBONYLIERUNG IM 2-PHASIGEN REAKTIONSSYSTEM**

(71) Anmelder: OQ Chemicals GmbH, 40789 Monheim am Rhein (DE)
(72) Erfinder: Schieweck, Benjamin, 46536 Dinslaken (DE); Louven, Yannik, 47906 Kempen (DE); Rahe, Jan Henry, 45701 Herten (DE)
(74) Vertreter: Michalski Hüttermann & Partner Patentanwälte mbB

(57) **Zusammenfassung**

Die vorliegende Erfindung betrifft ein Verfahren zur Herstellung aliphatischer Ester durch Alkoxycarbonylierung aliphatischer Olefine in Gegenwart von Kohlenmonoxid an einem Organophosphorliganden-modifizierten Metallkomplexkatalysator, wobei die aliphatischen Olefine in einem zweiphasigen Reaktionssystem aus einer wässrigen Phase mindestens umfassend wasserlösliche Metallkomplexkatalysatoren aus einem Übergangsmetall der 8. - 10. Nebengruppe und wasserlösliche Organophosphorliganden, und einer organischen Phase mindestens umfassend die aliphatischen Olefine und einen oder mehrere aliphatische C1 bis C8 Monoalkohole, zur Reaktion gebracht werden.

## Beschreibung

Die vorliegende Erfindung betrifft ein Verfahren zur Herstellung aliphatischer Ester durch Alkoxycarbonylierung aliphatischer Olefine in Gegenwart von Kohlenmonoxid an einem Organophosphorliganden-modifizierten Metallkomplexkatalysator, wobei die aliphatischen Olefine in einem zweiphasigen Reaktionssystem aus einer wässrigen Phase mindestens umfassend wasserlösliche Metallkomplexkatalysatoren aus einem Übergangsmetall der 8. - 10. Nebengruppe und wasserlösliche Organophosphorliganden, und einer organischen Phase mindestens umfassend die aliphatischen Olefine und einen oder mehrere aliphatische C1 bis C8 Monoalkohole, zur Reaktion gebracht werden.

Eine bekannte Reaktion zum Erhalt von Estern aus Olefinen ist die Alkoxycarbonylierung. In dieser Umsetzung werden ungesättigte Kohlenwasserstoffe mit Kohlenmonoxid und Alkohol nach folgendem allgemeinem Schema umgesetzt:

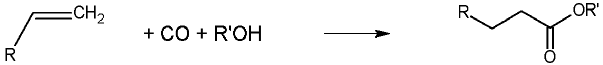

Die Reaktion erfolgt metallkatalysiert, wobei unterschiedlichste Metalle unter unterschiedlichsten Reaktionsbedingungen zum Einsatz kommen.

Auch in der Patentliteratur finden sich die unterschiedlichsten Lösungsvorschläge zur Durchführung von Alkoxycarbonylierungen.

So beschreibt beispielsweise die EP 4 011 895 A1 die Verwendung von Platin-Komplexen mit 1,2-substituierten benzylbasierten Diphosphinliganden für die Katalyse der Alkoxycarbonylierung ethylenisch ungesättigter Verbindungen.

Die EP 4 001 253 A1 beschreibt ein Verfahren zur doppelten Alkoxycarbonylierung von Dienen als Eintopfsynthese.

Die EP 4 011 894 A1 offenbart Platin-Komplexe mit Binaphthyl-Diphosphin-Liganden für die Katalyse der Hydroxycarbonylierung ethylenisch ungesättigter Verbindungen.

Derartige aus dem Stand der Technik bekannte Lösungen können noch weiteres Verbesserungspotential bieten. Dies bezieht sich insbesondere auf die Effizienz der Reaktion, den Energiebedarf der Umsetzung und die Vollständigkeit und Komplexität der Abtrennung des Metallkomplexkatalysators vom Produkt.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein Verfahren bereitzustellen, welches mit hohen Umsätzen und Ausbeuten auch schwierige Edukte umsetzen kann und welches nach erfolgter Reaktion ein energieschonendes und weitgehend vollständiges Abtrennen des eingesetzten Katalysators vom Produkt ermöglicht.

Die Lösung der Aufgabe erfolgt durch die Merkmale des unabhängigen Anspruchs, gerichtet auf das erfindungsgemäße Verfahren. Bevorzugte Ausgestaltungen der Erfindung sind in den Unteransprüchen, in der Beschreibung oder den Figuren angegeben, wobei weitere in den Unteransprüchen, in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Erfindungsgemäß gelöst wird die Aufgabe durch ein Verfahren zur Herstellung aliphatischer Ester durch Alkoxycarbonylierung aliphatischer Olefine in Gegenwart von Kohlenmonoxid an einem Organophosphorliganden-modifizierten Metallkomplexkatalysator, wobei die aliphatischen Olefine in einem zweiphasigen Reaktionssystem aus einer wässrigen Phase mindestens umfassend wasserlösliche Metallkomplexkatalysatoren aus einem Übergangsmetall der 8. - 10. Nebengruppe und wasserlösliche Organophosphorliganden, und einer organischen Phase mindestens umfassend die aliphatischen Olefine und einen oder mehrere aliphatische C1 bis C8 Monoalkohole, zur Reaktion gebracht werden.

Überraschend wurde gefunden, dass Alkoxycarbonylierungen unterschiedlichster aliphatischer Olefine sich sehr gut in heterogenen Reaktionssystemen aus einer getrennten wässrigen und einer getrennten organischen Phase durchführen lassen. Es ergeben sich auch mit schwierig umzusetzenden Edukten, beispielsweise mit einem oder mehreren zyklischen Strukturen und/oder einer oder mehreren Doppelbindungen, hohe Eduktumsätze und es werden mit geringen Katalysatorkonzentrationen mit hohen Reaktionsgeschwindigkeiten in hohen Ausbeuten die Olefine zu den entsprechenden Estern umgesetzt. Dies war nicht zu erwarten, da eine getrennte Verfahrensführung in einem zweiphasigen System, wobei der Katalysator über die wasserlöslichen Liganden in der wässrigen Phase und die organischen aliphatischen Olefine in der organischen, alkoholischen Phase vorliegen, eigentlich deutlich schlechtere Voraussetzungen für eine effiziente Reaktion zeigen sollten. Dies gilt insbesondere für sterisch gehinderte, mono- oder polyzyklische Olefin-Edukte mit einer oder mehreren Doppelbindungen. Zudem ergibt sich ein weiterer Vorteil darin, dass der Katalysator sehr einfach und sehr energieschonend von der Produktphase abtrennbar ist. Letzteres erspart insbesondere den Aufwand für energieintensive, thermische Trennoperationen, welche natürlich auch die Qualität der gebildeten Produkte belasten.

Das erfindungsgemäße Verfahren ist ein Verfahren zur Herstellung aliphatischer Ester durch Alkoxycarbonylierung aliphatischer Olefine in Gegenwart von Kohlenmonoxid an einem Organophosphorliganden-modifizierten Metallkomplexkatalysator. In der Reaktion werden nicht aromatische Kohlenwasserstoffe, welche an mindestens einer Stelle eine Doppelbindung tragen, umgesetzt. Durch die Reaktion werden am Molekül Estergruppen (COOR) eingeführt. Die Reaktion kann vorteilhafterweise unter Zugabe oder Anwesenheit einer Säure erfolgen. Es können auch Säuregemische zur Katalyse eingesetzt werden. Bei den optional einzusetzenden Säuren handelt es sich um Broenstedtsäuren, wobei beispielsweise Borsäuren oder deren Derivate, Salicylsäure, Phenylboransäure, Trifluormethansulfonsäure oder deren Derivate, Schwefelsäure, Toluolsulfonsäuren oder deren Derivate als geeignete Säuren eingesetzt werden können. Die Säuren können neben dem Metallkatalysator zur weiteren Katalyse der Umsetzung dienen. Neben den Edukten und optional der Säure wird zur Reaktion auch Kohlenmonoxid gegeben. Dies kann beispielsweise durch das Begasen oder durch Aufdrücken von CO auf das Reaktionssystems erfolgen. CO kann beispielsweise mit einem CO-Partialdruck im Bereich von 0,1 bis 10 MPa, bevorzugt von 1 bis 5 MPa, besonders bevorzugt von 3 bis 5 MPa zugeführt werden oder vorhanden sein. Hauptsächlich wird die Reaktionsgeschwindigkeit über einen Metallkomplexkatalysator gesteuert. Dieser weist zumindest ein Metall-Zentralatom und organische Liganden auf. Die organischen Liganden umfassen zumindest ein aromatisches oder ein aliphatisches Kohlenstoffwasserstoff-Grundgerüst und zusätzlich ein oder mehrere Phosphor-umfassende Substituenten.

In dem erfindungsgemäßen Verfahren werden aliphatische Olefine in einem zweiphasigen Reaktionssystem zur Reaktion gebracht. Die Alkoxycarbonylierung wird an aliphatischen Kohlenwasserstoffen durchgeführt, welche als lineare oder verzweigte Kette vorliegen können. Es ist aber auch möglich, dass die Olefine eine zyklische Gerüststruktur mit mindestens zwei, beispielsweise drei, vier oder fünf Ringen aufweisen. Die Ringe können eine gleiche oder unterschiedliche Ringgrößen aufweisen. Zudem weisen diese zyklischen Gerüststrukturen noch mindestens eine Doppelbindung auf. Es können im linearen oder zyklischen Grundgerüst auch mehrere Doppelbindungen vorliegen, wobei die Doppelbindungen im Grundgerüst kein aromatisches System ausbilden. Die Olefine werden insbesondere nicht in einer homogenen Reaktionsumgebung umgesetzt. Die Reaktionsumgebung umfasst mindestens zwei flüssige Phasen, welche sich unter Reaktionsbedingungen nicht oder nur partiell miteinander mischen und das Reaktionssystem ausbilden. Je nach Reaktionsführung, beispielsweise durch den Eintrag mechanischer Energie durch aktive Konvektion der beiden Phasen, kann es möglich sein, die Grenzoberfläche des zweiphasigen Systems zu erhöhen. Auch können die Reaktionsbedingungen so gewählt werden, dass eine höhere Mischbarkeit der beiden Phasen, beispielsweise durch Temperaturerhöhung, erreicht wird. Diese erhöhte Mischbarkeit ergibt sich aber nicht unter den Trennbedingungen, welche eine verbesserte und vollständigere Abtrennung der beiden Phasen durch eine schlechtere Mischbarkeit der Phasen erreicht.

Das zweiphasige Reaktionssystem umfasst eine wässrige Phase mindestens umfassend wasserlösliche Metallkomplexkatalysatoren aus einem Übergangsmetall der 8. - 10. Nebengruppe und wasserlösliche Organophosphorliganden. Der Metallkomplexkatalysator liegt in der wässrigen Phase der Reaktionsumgebung gelöst vor. Der Metallkomplexkatalysator umfasst zumindest ein Zentralatom aus einem Übergangsmetall. Geeignete Übergangsmetalle sind ausgesucht aus der Eisen-, Cobalt- oder Nickelgruppe. Als mögliche Metallzentren kommen also Eisen, Ruthenium, Osmium, Cobalt, Rhodium, Iridium, Nickel, Palladium, Platin in Frage. Der Metallkatalysator ist mit organischen Liganden komplexiert, wobei es sich bei den Liganden um wasserlösliche Liganden handelt. Das Ligandengrundgerüst basiert auf einem aliphatischem oder aromatischem, meist zyklischem Kohlenwasserstoffgerüst, wobei zusätzlich Phosphorsubstituenten am Kohlenwasserstoffgerüst vorhanden sind. Zusätzlich zu den funktionellen Phosphorgruppen weist das Kohlenwasserstoffgerüst ein oder mehrere Substituenten auf, welche die Wasserlöslichkeit des Liganden und dementsprechend des Komplexes bewirken. Dies kann beispielsweise durch Sulfongruppen am Kohlenwasserstoffgerüst erfolgen. Es ist aber auch möglich, die Wasserlöslichkeit durch andere funktionelle Substituenten, welche die Wasserlöslichkeit der Liganden erhöhen, bereitzustellen. Mögliche weitere Substituenten können beispielsweise ausgesucht sein aus der Gruppe bestehend aus Carbonsäure-, Amid- und Thiocarbonsäuregruppen oder Mischungen dieser. Es können eine oder mehrere dieser Substituenten am Kohlenwasserstoffgerüst vorhanden sein. Der Metallkomplexkatalysator ist wasserlöslich im Sinne der Erfindung, wenn dieser bei einer Temperatur von 20°C und einem pH-Wert von 7,0 +- 0,5 in Wasser eine Löslichkeit von größer oder gleich 10 g/L, bevorzugt größer oder gleich 20 g/L, weiterhin bevorzugt von größer oder gleich 50 g/L und besonders bevorzugt von größer oder gleich 100 g/L aufweist. Diese Löslichkeiten können ausreichende Umsetzungen und eine verbesserte Abtrennung des Katalysators nach der Reaktion bewirken.

Das zweiphasige Reaktionssystem umfasst eine organische Phase mindestens umfassend die aliphatischen Olefine und einen oder mehrere aliphatische C1 bis C8 Monoalkohole. Die organische Phase des Zweiphasensystems umfasst zumindest partiell die Edukte in Form der Monoalkohole und des Olefins. Die aliphatischen Olefine können beispielsweise C6 - C35 aliphatische Olefine sein. Bevorzugt können die aliphatischen Olefine eine C-Anzahl im Bereich von C8 bis C30, weiter bevorzugt von C8 bis C20 aufweisen. Bevorzugt können diese polyzyklischen aliphatischen Olefine ein, zwei oder drei Doppelbindungen tragen. Bevorzugt können die Monoalkohole C2 bis C8, weiter bevorzugt C3 bis C8 Monoalkohole sein. Im Falle sehr kurzkettiger Alkohole können diese unter Reaktionsbedingungen auch zum Teil in der Wasserphase vorliegen. In diesem Fall wird die organische Phase allein oder zum überwiegenden Anteil aus dem aliphatischen Olefin gebildet.

In einer bevorzugten Ausführungsform des Verfahrens kann die wässrige Phase und das Olefin in einem Gewichtsverhältnis, berechnet nach Gewicht wässrige Phase dividiert durch Gewicht Olefin, von größer oder gleich 1 und kleiner oder gleich 10 zueinander vorliegen. Dieses Gewichtsverhältnis zwischen wässriger und organischer Phase hat sich zum Erhalt hoher Umsätze als besonders geeignet herausgestellt. Das Gewicht der wässrigen Phase umfasst das Gewicht des Wassers sowie der weiteren wasserlöslichen Substanzen. Das Gewicht beinhaltet also das Wasser, das Gewicht des wasserlöslichen Komplexkatalysators sowie der Säure, falls diese denn wasserlöslich ist. Eine Teillöslichkeit der Alkohole in der wässrigen Phase bleibt zur Berechnung des Verhältnisses unberücksichtigt. Das Gewicht der eingesetzten Alkohole wird also rechnerisch immer zur organischen Phase zugeschlagen. Kleinere Gewichtsverhältnisse als 1 können nachteilig sein, da ein nicht ausreichendes Volumen an wässriger Phase für eine schnelle Reaktion bereitsteht. Höhere Verhältnisse als 10 können nachteilig sein, da in diesem Fall die Reaktionszeiten aufgrund einer zu geringen Olefinkonzentration zum Übertritt in die wässrige Phase unnötig verlängert werden. Bevorzugt kann das Verhältnis größer oder gleich 1,5 und kleiner oder gleich 9 und weiterhin bevorzugt größer oder gleich 1,75 und kleiner oder gleich 8 betragen.

Innerhalb einer weiter bevorzugten Ausgestaltung des Verfahrens kann die wässrige Phase und das Olefin in einem Gewichtsverhältnis, berechnet nach Gewicht wässrige Phase dividiert durch Gewicht Olefin, von größer oder gleich 2 und kleiner oder gleich 5 zueinander vorliegen. Bedingt durch die Zweiphasigkeit des Reaktionssystems hat sich oben angegebenes Gewichtsverhältnis zwischen den beiden Phasen als besonders geeignet herausgestellt. Es ist im System eine hinreichende Menge an wässriger Phase vorhanden, wobei auch das Volumen der organische Olefinphase hoch genug ist, um einen genügenden Übertritt der Olefine in die wässrige Phase zu gewährleisten. Durch dieses Verhältnis können insbesondere die Umsatzgeschwindigkeiten und somit generell die Umsätze in der Reaktion beeinflusst werden. Im angegebenen Verhältnisbereich werden besonders bevorzugte, hohe Eduktumsätze erhalten.

Innerhalb eines weiter bevorzugten Aspektes des Verfahrens können die aliphatischen Alkohole aus der Gruppe bestehend aus n-Propanol, n-Butanol und Mischungen daraus ausgewählt sein. Insbesondere bei den mittelkettigen Alkoholen aus der erfindungsgemäß beanspruchten Alkoholgruppe kann sich ein besonders bevorzugtes Verhältnis aus begrenzter Mischbarkeit mit der wässrigen Phase und generelle Phasentrennung zur wässrigen Phase ergeben. Mit diesen Alkoholen werden sehr gute Umsetzungskinetiken zu den Estern erhalten und es erfolgt nach der Reaktion nur ein sehr geringer Übertritt der Metallkomplexkatalysatoren in die organische Phase.

Nach einer bevorzugten Charakteristik des Verfahrens kann der Metallkatalysator bezogen auf die wässrige Phase in einer Konzentration von größer oder gleich 0,01 mol% und kleiner oder gleich 1 mol% in der Reaktionslösung vorliegen. In einem zweiphasigen System lassen sich auch mit relativ geringen Katalysatorkonzentrationen ausreichende Umsetzungsgeschwindigkeiten, gute Umsätze und Ausbeuten erzielen. Dies gilt insbesondere für eher schwer umzusetzende, zyklische oder polyzyklische Verbindungen mit einer oder mehreren Doppelbindungen.

Innerhalb eines bevorzugten Aspektes des Verfahrens kann das molare Verhältnis von Katalysatormetall zu Olefin, ausgedrückt als mol Katalysatormetall dividiert durch mol Olefin, größer oder gleich 0,0001 und kleiner oder gleich 0,1 betragen. Trotz der Phasentrennung zwischen Katalysatormetall und Edukt können mit relativ geringen Katalysatormengen auch schwierig umzusetzende, zyklische Edukte mit guten Umsätzen und Ausbeuten zu Estern reagieren. Bevorzugt kann das Verhältnis weiterhin größer oder gleich 0,001 und kleiner oder gleich 0,02, weiter bevorzugt größer oder gleich 0,002 und kleiner oder gleich 0,01 betragen.

In einer weiter bevorzugten Ausführungsform des Verfahrens kann das molare Einsatzverhältnis des wasserlöslichen Organophosphorliganden zum Katalysatormetall, ausgedrückt als mol Ligand dividiert durch mol Katalysatormetall, größer oder gleich 1 und kleiner oder gleich 40 betragen. Die Umsetzungen lassen sich auch in einem relativ engen Bereich an wasserlöslichen Liganden über lange Zeiträume verlässlich steuern. Ohne durch die Theorie gebunden zu sein, scheint die Katalysatordeaktivierung in einem zweiphasigen System im Vergleich zu einphasigen Umsetzungen auch mit geringen Ligandenmengen über längere Zeiträume beherrschbarer zu sein. Bevorzugt kann das Verhältnis größer oder gleich 2 und kleiner oder gleich 30, weiter bevorzugt größer oder gleich 3 und kleiner oder gleich 25 betragen.

In einer weiter bevorzugten Ausführungsform des Verfahrens kann der pH-Wert der wässrigen Phase größer oder gleich pH 0 und kleiner gleich pH 2,0 betragen. Innerhalb diese pH-Wertebereiches lassen sich in der wässrigen Phase hohe Umsätze und hohe Ausbeuten an Estern, auch für schwierig umzusetzende, mono- oder polyzyklische Edukte, erreichen.

In einer weiteren Ausgestaltung des Verfahrens kann nach der Reaktion die organische Produktphase von der wässrigen Katalysatorphase mechanisch abgetrennt werden. Ein weiterer Vorteil der Verfahrensführung kann darin bestehen, dass der Katalysator ohne großen Energieaufwand einfach und effizient von der Produktphase getrennt werden kann. Dies kann beispielsweise durch ein Absetzen und mechanisches Dekantieren der Phasen nach dem Abkühlen erfolgen. Diese Form der Abtrennung ist im Vergleich zu einer thermischen Abtrennung, beispielsweise durch Destillation, deutlich energie- und produktschonender.

Im Rahmen einer weiterhin bevorzugten Ausgestaltung des Verfahrens können die aliphatischen Monoalkohole C1-C3 aliphatische Monoalkohole sein und zu der wässrigen und der organischen Phase kann ein weiteres unpolares Lösungsmittel zugegeben werden. Im Falle des Einsatzes von Alkoholen mit sehr niedriger C-Zahl kann es vorteilhaft sein, dass zum System ein weiteres, unpolares und damit nicht oder ein nur gering wasserlösliches Lösemittel gegeben wird. Durch die Zugabe kann das Zweiphasensystem stabilisiert und das Volumen der organischen Phase vergrößert werden. Durch die Zugabe des Lösemittels kann zudem ein verbesserter Übertritt der organischen Edukte in die wässrige Phase erfolgen, sodass sich verbesserte Umsätze und Ausbeuten ergeben. Ein weiterer Vorteil kann darin bestehen, dass sich das Produkt in der Phasentrennung nach der Reaktion besser vom Katalysator trennt. Ein unpolares Lösungsmittel ist ein Lösungsmittel welches eine Löslichkeit in Wasser bei 20°C von kleiner oder gleich 100 g/L aufweist, bevorzugt kann das Lösungsmittel eine Löslichkeit in Wasser bei 20°C von kleiner oder gleich 70 g/L, des Weiteren bevorzugt von kleiner oder gleich 30 g/L aufweisen.

Im Rahmen einer weiterhin bevorzugten Ausgestaltung des Verfahrens kann das weitere Lösungsmittel aus der Gruppe bestehend aus aliphatischen C5 bis C10 Kohlenwasserstoffen, aliphatischen C4 bis C10 Ether und Mischungen dieser ausgewählt sein. Diese Gruppe an unpolaren Lösemitteln kann insbesondere dazu beitragen, dass im Zweiphasensystem, unter Verwendung relativ wasserlöslicher Alkohole, sich ein stabileres Zweiphasensystem mit ausreichenden Volumina der organischen und wässrigen Phase ergeben. Über den Zusatz dieser Gruppe an Lösemitteln lassen sich zudem besonders hohe Trennleistungen bezüglich der Katalysator- und Produktphase nach der Reaktion erhalten.

In einer bevorzugten Ausführungsform des Verfahrens kann das weitere Lösungsmittel in einem Gewichtsanteil von größer oder gleich 10 Gew. % und kleiner oder gleich 50 Gew. % bezogen auf das Gewicht des Olefins zugegeben werden. Zur Unterstützung und Stabilisierung der Ausbildung ausreichender Volumina der organischen Phase hat es sich als vorteilhaft herausgestellt, dass nur relativ geringe Mengen an unpolaren Lösemitteln zum System hinzugegeben werden. Diese Zugabe kann insbesondere eine einfachere Abtrennung des Katalysators von der Produktphase ermöglichen.

Innerhalb einer weiter bevorzugten Ausgestaltung des Verfahrens können die wasserlöslichen Organophosphorliganden aus der Gruppe der sulfonierten Monophosphine, der sulfonierten Diphosphine und Mischungen dieser ausgewählt sein. Insbesondere der Einsatz dieser sulfonierten Liganden kann zur Ausbildung eines effizienten, wasserlöslichen Katalysatorsystems beitragen, welches sich durch hohe Umsätze und Ausbeuten auch für schwer umzusetzende Edukte auszeichnet. Zudem ergeben sich durch den Einsatz dieser Liganden im Zweiphasensystem dieser Umsetzung hohe Standzeiten mit einer nur geringen thermischen Deaktivierung des Katalysators sowie eine sehr gute Abtrennung vom organischen Produkt nach Reaktionsende. Zu der Gruppe der sulfonierten Monophosphine gehört beispielsweise Trinatrium-3,3',3"-phosphintriyltribenzolsulfonat. Zu der Gruppe der sulfonierten Diphosphine gehört beispielsweise Dinatrium 4,5-Bis(diphenylphosphino)-9,9-dimethyl-2,7-disulfoxanthen oder dessen Derivate mit unterschiedlichen Anzahlen an Sulfo-Gruppen.

Innerhalb einer weiter bevorzugten Ausgestaltung des Verfahrens kann das aliphatische Olefin ein polyzyklisches aliphatisches Olefin sein. Mittels des erfindungsgemäßen Verfahrens lassen sich auch sterisch herausfordernde, polyzyklische Olefine umsetzen.

Auch Edukte mit einem starren, polyzyklischen Ringgerüst lassen sich mit hohen Ausbeuten im erfindungsgemäßen Verfahren umsetzen.

Innerhalb eines weiter bevorzugten Aspektes des Verfahrens kann das aliphatische Olefin ein polyzyklisches aliphatisches Diolefin sein. Mittels des erfindungsgemäßen Verfahrens lassen sich auch sterisch sehr herausfordernde polyzyklische Olefine mit mehreren Olefingruppen erfolgreich umsetzen. Dies bedeutet, dass selbst Edukte mit einem starren, polyzyklischen Ringgerüst und mehreren Doppelbindungen sich mit hohen Ausbeuten mehrfach umsetzen lassen. So ist beispielsweise die einmalige oder doppelte Umsetzung von Dicyclopentadien innerhalb der erfindungsgemäßen, zweiphasigen Alkoxycarbonylierung mit hohen Edukt-Umsätzen und Ester-Ausbeuten möglich.

Weitere Einzelheiten, Merkmale und Vorteile des Gegenstandes der Erfindung ergeben sich aus den Unteransprüchen sowie aus den nachfolgenden Beispielen.

### Beispiele

### I. Alkoxycarbonylierungen unter Verwendung eines TPPTS-Liganden-Metallkomplexes

Es wird eine zweiphasige Alkoxycarbonylierung an einem polyzyklischen aliphatischen Diolefin unter Erhalt polyzyklischer aliphatischer Diester durchgeführt.

Für sämtliche Versuche werden die Einsatzstoffe zusammen in einem Autoklav vorgelegt. Als polyzyklisches aliphatisches Diolefin wird Dicyclopentadien eingesetzt. Als Katalysator-Precursor wird Pd(acac)₂ und als wasserlöslicher Ligand Trinatrium-3,3',3"-phosphintriyltribenzolsulfonat (TPPTS) nach der folgenden Formel: 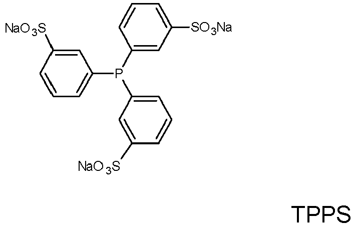 eingesetzt. Die Katalysatorkonzentration beträgt bei sämtlichen Versuchen 0,5 mol% bezogen auf die eingesetzte Olefinmenge. Die Ligandenkonzentration ist in Abhängigkeit der Metallkonzentration angegeben. Die Säurekonzentration ist in Abhängigkeit der Olefinkonzentration angegeben Der Autoklav wird verschlossen und 3-mal mit CO-gespült. Der gewünschte CO-Druck von 40 bar wird angelegt und der Autoklav auf die angegebene Temperatur aufgeheizt. Danach wird der Versuch für 6 h bei 120 °C durchgeführt. Dabei wird der Druck im System kontinuierlich mit CO auf 40 bar nachgeregelt. Nach der Reaktion wird das Gesamtsystem entnommen, auf Raumtemperatur abgekühlt und die Phasen getrennt. Die organische Phase wird zur Ermittlung der Umsätze und Ausbeuten mittels GC-Analyse untersucht.

Die variablen Versuchsbedingungen, die Umsätze und die erhaltenen Ausbeuten ergeben sich wie folgt:

| Nummer | 1 | 2 | 3 | 4 | 5 | 6 | 7 | 8 | 9 | 10 | 11 |
|---|---|---|---|---|---|---|---|---|---|---|---|
| | | | | | | | | | | | |
| Alkohol | Methanol | | | | | | | n-Butanol | | | |
| Säure | H2SO4 | | MSA | H2SO4 | MSA | p- TSA | | H2SO4 | MSA | p- TSA | MSA |
| c(Ligand) [mol%] | 5 | 15 | | 5 | | | | | | | |
| c(H+) [mol%] | 5 | 25 | | 5 | | | | | | | |
| C(OH) [Äq.] | 10 | 5 | | 4 | | | | | | | |
| | | | | | | | | | | | |
| Umsatz [%] | 99 | 98 | 99 | 97 | 97 | 97 | 98 | 98 | 98 | 99 | 99 |

| Ausbeuten in % | | | | | | | | | | | |
|---|---|---|---|---|---|---|---|---|---|---|---|
| Mono | 40 | 29 | 32 | 49 | 58 | 77 | 72 | 41 | 72 | 69 | 72 |
| Di | 44 | 56 | 54 | 39 | 31 | 12 | 17 | 4 | 3 | 13 | 12 |
| Gesamt | 84 | 85 | 86 | 88 | 89 | 90 | 89 | 45 | 75 | 81 | 84 |

| | | | | | | | | | | | |
|---|---|---|---|---|---|---|---|---|---|---|---|
| MSA=Methansulfonsäure, p-TSA=para-Toluolsulfonsäure, Mono = Monoester, Di = Diester, Gesamt = Ausbeute Monoester+ Ausbeute Diester. | | | | | | | | | | | |

Die Versuche belegen, dass man Alkoxycarbonylierung aliphatischer Olefine mit hohen Umsätzen und hohen Ausbeuten auch mit schwer umzusetzenden polyzyklischen Olefinen mit mehreren Doppelbindungen in einem zweiphasigen Reaktionssystem durchführen kann.

### II. Alkoxycarbonylierungen unter Verwendung eines Sulfoxantphos -Liganden-Metallkomplexes

Es wird eine zweiphasige Alkoxycarbonylierung an einem polyzyklischen aliphatischen Diolefin unter Erhalt polyzyklischer aliphatischer Diester durchgeführt.

Für sämtliche Versuche werden die Einsatzstoffe zusammen in einem Autoklav vorgelegt. Als polyzyklisches aliphatisches Diolefin wird Dicyclopentadien eingesetzt. Als Katalysator-Precursor wird Pd(acac)₂ und als wasserlöslicher Ligand (Sulfoxantphos) nach der folgenden Formel: 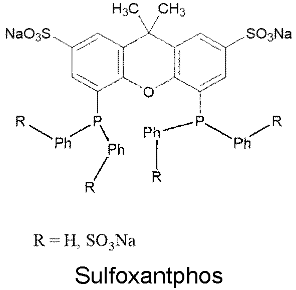 eingesetzt. Der eingesetzte Katalysator kann bevorzugt mindestens 3, bevorzugt mindestens 4 und weiter bevorzugt mindestens 5 Sulfo-Gruppen tragen. Die Katalysatorkonzentration beträgt bei sämtlichen Versuchen 0,5 mol% bezogen auf die eingesetzte Olefinmenge. Der Autoklav wird verschlossen und 3-mal mit CO-gespült. Der gewünschte CO-Druck wird angelegt und der Autoklav auf die angegebene Temperatur aufgeheizt. Danach wird der Versuch für 6 h bei 120 °C durchgeführt. Dabei wird der Druck im System kontinuierlich mit CO nachgeregelt. Nach der Reaktion wird das Gesamtsystem entnommen, auf Raumtemperatur abgekühlt und die Phasen getrennt. Die organische Phase wird zur Ermittlung der Umsätze und Ausbeuten mittels GC-Analyse untersucht.

Die variablen Versuchsbedingungen, die Umsätze und die erhaltenen Ausbeuten ergeben sich wie folgt:

| Nummer | 12 | 13 | 14 | 15 | 16 | 17 |
|---|---|---|---|---|---|---|
| Alkohol | Meth | Eth | n-Prop | n-But | n-Prop | n-Prop |
| Säure | MSA | | | | | HSO4 |
| c(Ligand) [mol%] | 1 | | | | | 2 |
| c(H+) [mol%] | 25 | | | | | |
| C(OH) [Äq.] | 10 | | | | | |
| m(MCH)/m(DCPD) | 0,74 | | | | - | - |
| bar | 50 | | | | | 80 |
| | | | | | | |
| Umsatz [%] | 100% | 99% | 99% | 100% | 100% | 99% |

| Ausbeuten in % | | | | | | |
|---|---|---|---|---|---|---|
| Mono | 1% | 5% | 14% | 51% | 1% | 5% |
| Di | 39% | 41% | 49% | 23% | 90% | 86% |
| Gesamt | 40% | 46% | 63% | 74% | 91% | 90% |

| | | | | | | |
|---|---|---|---|---|---|---|
| Meth=Methanol, Eth=Ethanol, n-Prop = n-Propanol, n-But = n-Butanol, MCH = Methylcyclohexan, Mono = Monoester + Monosäure, Di = Diester + Disäure, Gesamt = Ausbeute Monoester+ Ausbeute Diester. | | | | | | |

Die Versuche belegen, dass man Alkoxycarbonylierung aliphatischer Olefine mit hohen Umsätzen und hohen Ausbeuten auch mit schwer umzusetzenden polyzyklischen Olefinen mit mehreren Doppelbindungen in einem zweiphasigen Reaktionssystem mittels Sulfoxanthphos-Metallkomplexen durchführen kann.

## Patentansprüche

1. Verfahren zur Herstellung aliphatischer Ester durch Alkoxycarbonylierung aliphatischer Olefine in Gegenwart von Kohlenmonoxid an einem Organophosphorliganden-modifizierten Metallkomplexkatalysator, **dadurch gekennzeichnet, dass** die aliphatischen Olefine in einem zweiphasigen Reaktionssystem aus einer wässrigen Phase mindestens umfassend wasserlösliche Metallkomplexkatalysatoren aus einem Übergangsmetall der 8. - 10. Nebengruppe und wasserlösliche Organophosphorliganden, und einer organischen Phase mindestens umfassend die aliphatischen Olefine und einen oder mehrere aliphatische C1 bis C8 Monoalkohole, zur Reaktion gebracht werden.

2. Verfahren nach Anspruch 1, wobei die wässrige Phase und das Olefin in einem Gewichtsverhältnis, berechnet nach Gewicht wässrige Phase dividiert durch Gewicht Olefin, von größer oder gleich 1 und kleiner oder gleich 10 zueinander vorliegen.

3. Verfahren nach einem der vorhergehenden Ansprüche, wobei die wässrige Phase und das Olefin in einem Gewichtsverhältnis, berechnet nach Gewicht wässrige Phase dividiert durch Gewicht Olefin, von größer oder gleich 2 und kleiner oder gleich 5 zueinander vorliegen.

4. Verfahren nach einem der vorhergehenden Ansprüche, wobei die aliphatischen Alkohole ausgewählt sind aus der Gruppe bestehend aus n-Propanol, n-Butanol und Mischungen daraus.

5. Verfahren nach einem der vorhergehenden Ansprüche, wobei der Metallkatalysator bezogen auf die wässrige Phase in einer Konzentration von größer oder gleich 0,01 mol% und kleiner oder gleich 1 mol% in der Reaktionslösung vorliegt.

6. Verfahren nach einem der vorhergehenden Ansprüche, wobei das molare Verhältnis von Katalysatormetall zu Olefin, ausgedrückt als mol Katalysatormetall dividiert durch mol Olefin, größer oder gleich 0,0001 und kleiner oder gleich 0,1 beträgt.

7. Verfahren nach einem der vorhergehenden Ansprüche, wobei das molare Einsatzverhältnis des wasserlöslichen Organophosphorliganden zum Katalysatormetall, ausgedrückt als mol Ligand dividiert durch mol Katalysatormetall, größer oder gleich 1 und kleiner oder gleich 40 beträgt.

8. Verfahren nach einem der vorhergehenden Ansprüche, wobei der pH-Wert der wässrigen Phase größer oder gleich pH 0 und kleiner gleich pH 2,0 beträgt.

9. Verfahren nach einem der vorhergehenden Ansprüche, wobei nach der Reaktion die organische Produktphase von der wässrigen Katalysatorphase mechanisch abgetrennt wird.

10. Verfahren nach einem der vorhergehenden Ansprüche, wobei die aliphatischen Monoalkohole C1-C3 aliphatische Monoalkohole sind und zu der wässrigen und der organischen Phase ein weiteres unpolares Lösungsmittel zugegeben wird.

11. Verfahren nach Anspruch 10, wobei das weitere Lösungsmittel ausgewählt ist aus der Gruppe bestehend aus aliphatischen C5 bis C10 Kohlenwasserstoffen, aliphatischen C4 bis C10 Ether und Mischungen dieser.

12. Verfahren nach einem der Ansprüche 10 bis 11, wobei das weitere Lösungsmittel in einem Gewichtsanteil von größer oder gleich 10 Gew. % und kleiner oder gleich 50 Gew. % bezogen auf das Gewicht des Olefins zugegeben wird.

13. Verfahren nach einem der vorhergehenden Ansprüche, wobei die wasserlöslichen Organophosphorliganden ausgewählt sind aus der Gruppe der sulfonierten Monophosphine, der sulfonierten Diphosphine und Mischungen dieser.

14. Verfahren nach einem der vorhergehenden Ansprüche, wobei das aliphatische Olefin ein polyzyklisches aliphatisches Olefin ist.

15. Verfahren nach einem der vorhergehenden Ansprüche, wobei das polyzyklische aliphatische Olefin ein polyzyklisches aliphatisches Diolefin ist.
